(19) Europäisches Patentamt

European Patent Office

Office européen des brevets

(11) **EP 1 182 246 A1**

(12) **DEMANDE DE BREVET EUROPEEN**

(43) Date de publication:
**27.02.2002 Bulletin 2002/09**

(51) Int Cl.7: **C10G 25/03**, C10G 67/06

(21) Numéro de dépôt: **01402164.6**

(22) Date de dépôt: **13.08.2001**

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **25.08.2000 FR 0010974**

(71) Demandeur: **INSTITUT FRANCAIS DU PETROLE 92852 Rueil-Malmaison Cedex (FR)**

(72) Inventeurs:
• **Jolimaitre, Elsa**
  **75011 Paris (FR)**
• **Ducreux, Olivier**
  **78380 Bougival (FR)**

(74) Mandataire: **Andréeff, François et al
Département Brevets, Institut Français du
Petrole, 1 & 4 avenue de Bois-Préau
92852 Rueil Malmaison (FR)**

(54) **Procédé de séparation de paraffines multibranches utilisant un adsorbant zeolithique de structure mixte**

(57)  On décrit un procédé de séparation de paraffines multibranchées, comprises dans une charge hydrocarbonée contenant des hydrocarbures ayant de 5 à 8 atomes de carbone par molécule, comprenant au moins une unité de séparation fonctionnant par adsorption et contenant au moins un adsorbant zéolithique de structure mixte avec des canaux principaux dont l'ouverture est définie par un anneau à 10 atomes d'oxygène et des canaux secondaires dont l'ouverture est définie par un anneau à au moins 12 atomes d'oxygène, les canaux secondaires n'étant accessibles à la charge à séparer que par les canaux principaux.

Les adsorbants zéolithiques visés par l'invention sont des zéolithes qui appartiennent particulièrement aux types structuraux EUO, NES et MWW. Les zéolithes NU-85 et NU-86 sont également particulièrement adaptées à la mise en oeuvre du procédé de l'invention.

EP 1 182 246 A1

**Description**

L'invention concerne un procédé de séparation de paraffines multibranchées mettant en oeuvre au moins une unité de séparation fonctionnant par adsorption dans laquelle l'adsorbant est un solide zéolithique microporeux présentant une structure mixte avec des canaux de tailles distinctes.

L'invention vise particulièrement le domaine de l'isomérisation des essences de façon à améliorer leur indice d'octane. En effet, du point de vue de l'indice d'octane, il est préférable que les hydrocarbures constituant l'essence soient les plus ramifiés possible. Ainsi une essence contenant des diméthylbutanes présente un meilleur indice d'octane qu'une essence contenant des méthylpentanes.

Pour augmenter l'indice d'octane d'une essence, plusieurs techniques ont déjà été proposées. Dans un premier temps, les composés aromatiques, constituants principaux des essences de réformage, les isoparaffines produites par alkylation aliphatique ou isomérisation d'essences légères ont compensé la perte d'indice d'octane résultant de la suppression du plomb dans les essences, cette suppression étant due à la prise en compte de contraintes environnementales toujours plus drastiques. Par la suite, des composés oxygénés tels que le Méthyl Tertiobutyl Ether (MTBE) ou l'Ethyl Tertiobutyl Ether (ETBE) ont été introduits dans les carburants. Plus récemment, la toxicité reconnue de composés tels que les aromatiques, en particulier le benzène, les oléfines et les composés soufrés, ainsi que la volonté de diminuer la pression de vapeur des essences, ont entraîné la production d'essences reformulées. Par exemple, depuis le premier janvier 2000, les teneurs maximales en oléfines, composés aromatiques totaux et benzène dans les essences distribuées en France sont respectivement de 18 % vol., 42 % vol. et 1 % vol.

Les «pools essence» comprennent plusieurs composants. Les composants majoritaires sont l'essence de réformage, qui comprend habituellement entre 60 et 80 % vol. de composés aromatiques, et les essences de craquage catalytique (FCC), qui contiennent typiquement 35 % vol. d'aromatiques mais apportent la majorité des composés oléfiniques et soufrés présents dans les essences. Les autres composants peuvent être les alkylats, sans composés aromatiques ni oléfiniques, les essences légères isomérisées ou non isomérisées, qui ne contiennent pas de composés insaturés, les composés oxygénés tels que le MTBE et des butanes. Dans la mesure où les teneurs maximales en aromatiques ne sont pas réduites en dessous de 30 ou 40 % vol., la contribution des réformats dans les «pools essence» restera importante, typiquement 40 % vol. Une sévérisation accrue de la teneur maximale admissible en composés aromatiques à 20 ou 25 % vol. entraînera une diminution de l'utilisation du réformage, et par voie de conséquence la nécessité de valoriser les coupes C7-C10 de distillation directe par d'autres voies que le réformage. Leur valorisation par hydro-isomérisation est une des voies possibles comme décrit dans la demande de brevet ayant pour titre « Procédé associant hydro-isomérisation et séparation avec un adsorbant zéolithique à structure mixte pour la production d'essences à haut indice d'octane » déposée le même jour par la Demanderesse. Le procédé d'hydro-isomérisation conduit à la formation de composés multibranchés à partir des composés de faibles indices d'octane. Il ne peut être mis en oeuvre qu'à la condition de recycler les paraffines linéaires et monobranchées en C6-C10, puisque la réaction d'hydro-isomérisation est équilibrée et que ces paraffines de faibles indices d'octane ne peuvent pas être envoyées vers le «pool essence».

**Etat de la technique antérieure**

Pour augmenter le taux de paraffines multibranchées comprises dans une charge issue d'une zone d'isomérisation, on peut employer des tamis moléculaires sélectifs grâce à la dimension des pores accessibles.

La séparation par adsorption des paraffines linéaires, monobranchées et multibranchées peut être effectuée par deux techniques différentes biens connues de l'homme de l'art : la séparation par différence de thermodynamique d'adsorption et la séparation par différence de cinétiques d'adsorption des espèces à séparer. Selon la technique utilisée, l'adsorbant choisi aura des diamètres de pores différents. Les zéolithes, composées de canaux, sont des adsorbants de choix pour réaliser la séparation de telles paraffines.

Le terme diamètre de pore est conventionnel pour l'homme du métier. Il est utilisé pour définir de façon fonctionnelle la taille d'un pore en terme de taille de molécule capable d'entrer dans ce pore. Il ne désigne pas la dimension réelle du pore car celle-ci est souvent difficile à déterminer puisque souvent de forme irrégulière (c'est-à-dire non circulaire). D.W. Breck fournit une discussion sur le diamètre de pore effectif dans son livre intitulé *Zeolite Molecular Sieves* (John Wiley and Sons, New York, 1974) aux pages 633 à 641. Les sections des canaux des zéolithes étant des anneaux d'atomes d'oxygènes, on peut également définir la taille des pores des zéolithes par le nombre d'atomes d'oxygène formant la section annulaire des anneaux, désigné par le terme « member rings » ou MR en anglais. Il est par exemple indiqué dans l'« Atlas of Zeolite Structure Types » (W.M. Meier et D.H. Olson, 4ème Edition, 1996) que les zéolithes de type structural FAU ont un réseau de canaux cristallins de 12 MR c'est à dire dont la section est constituée de 12 atomes d'oxygène. Cette définition est bien connue de l'homme de l'art et sera utilisée par la suite.

Dans le cas de la séparation dite «thermodynamique», l'adsorbant a un diamètre de pores supérieur au diamètre critique des molécules à séparer. Ainsi, quelques brevets décrivent la séparation des paraffines multibranchées des paraffines linéaires et monobranchées par adsorption thermodynamiquement sélective. Le brevet US-A-5,107,052 propose d'adsorber préférentiellement les paraffines multibranchées sur des zéolithes SAPO-5, AlPO-5, SSZ-24, MgAPO-5 ou MAPSO-5. Le brevet US-A-3,706,813 propose le même type de sélectivité sur des zéolithes X ou Y échangées au baryum. Le brevet US-A-6,069,289 propose au contraire d'utiliser des zéolithes ayant des sélectivités inversement proportionnelles au degré de branchement des paraffines, telles que les zéolithes beta, X ou Y échangées avec des cations alcalins ou alcalino-terreux, SAPO-31, MAPO-31. Toutes les zéolithes citées précédemment ont des diamètres de pores de 12 MR.

Dans le cas de la séparation dite « diffusionnelle », le pouvoir séparateur de l'adsorbant est dû à la différence de cinétique de diffusion des molécules à séparer dans les pores de la zéolithe. Dans le cas de la séparation des paraffines multibranchées des paraffines monobranchées et linéaires, on peut ainsi utiliser le fait que plus le degré de branchement est important, plus le diamètre cinétique de la molécule augmente, et donc plus la cinétique de diffusion est faible. Pour que l'adsorbant puisse avoir un pouvoir de séparation, l'adsorbant doit avoir un diamètre de pore proche de celui des molécules à séparer, ce qui correspond aux zéolithes dont le diamètre des pores est de 10 MR. De nombreux brevets décrivent la séparation des paraffines linéaires, monobranchées et multibranchées par sélectivité diffusionnelle. Les brevets US-A-4,717,784, US-A-4,804,802, US-A-4,855,529 et US-A-4,982,048 utilisent des adsorbants de taille de canaux intermédiaires entre 8 et 10 MR, l'adsorbant préféré étant la ferriérite. Le brevet US-A-4,982,052 préconise l'utilisation de la silicalite. Les brevets US-A-4,956,521, US-A-5,055,633 et US-A-5,055,634 décrivent l'utilisation de zéolithes possédant des pores de section elliptique de dimensions comprises entre 5,0 et 5,5 Å suivant le petit axe et environ 5,5 à 6,0 Å suivant le grand axe, et en particulier la ZSM-5 et sa forme désaluminée, la silicalite, ou de dimensions comprises entre 4,5 et 5,0 Å, et en particulier la ferriérite, la ZSM-23 et la ZSM-11.

Les adsorbants zéolithiques proposés pour la séparation diffusionnelle des paraffines multibranchées présentent une structure homogène quant à leur taille de canaux et ne sont composés de canaux que de faible taille (8 à 10 MR) ce qui réduit considérablement leur capacité volumique d'adsorption. Ces matériaux qui pèchent notamment par leur faible capacité d'adsorption ne permettent pas d'obtenir une efficacité optimale du procédé de séparation.

## Résumé de l'invention

La présente invention est basée sur l'utilisation nouvelle d'adsorbants zéolithiques à structure mixte, composée de deux types de canaux de taille distincte, dans un procédé de séparation de paraffines multibranchées comprises dans une charge hydrocarbonée contenant des hydrocarbures ayant de 5 à 8 atomes de carbone par molécule, notamment des paraffines linéaires, monobranchées et multibranchées. Le procédé de l'invention est tel qu'il comprend au moins une unité de séparation fonctionnant par adsorption et contenant au moins un adsorbant zéolithique de structure mixte avec des canaux principaux dont l'ouverture est définie par un anneau à 10 atomes d'oxygène (également appelés 10 MR) et des canaux secondaires dont l'ouverture est définie par un anneau à au moins 12 atomes d'oxygène (12 MR), les canaux à au moins 12 MR n'étant accessibles à la charge à séparer que par les canaux à 10 MR.

Les adsorbants zéolithiques visés par l'invention sont des zéolithes qui appartiennent avantageusement aux types structuraux EUO, NES et MWW. Les zéolithes NU-85 et NU-86 sont également particulièrement adaptées à la mise en oeuvre du procédé de l'invention.

## Intérêt de l'invention

Les adsorbants zéolitiques utilisés dans la mise en oeuvre du procédé de l'invention présentent des propriétés adsorbantes nettement améliorées par rapport aux adsorbants de l'art antérieur, notamment en ce qui concerne la capacité d'adsorption elle-même. En effet, il a été découvert, de manière surprenante, que l'utilisation d'un adsorbant zéolithique présentant au moins deux types de canaux de taille distincte, des canaux principaux dont l'ouverture est définie par un anneau à 10 atomes d'oxygène et des canaux secondaires dont l'ouverture est définie par un anneau à au moins 12 atomes d'oxygène, a un effet bénéfique sur les performances d'un procédé de séparation de paraffines multibranchées comprises dans une charge hydrocarbonée contenant des hydrocarbures ayant de 5 à 8 atomes de carbone par molécule. L'adsorbant zéolithique utilisé dans le procédé de l'invention allie une bonne sélectivité à une capacité d'adsorption optimale, permettant notamment d'assurer des gains de productivité par rapport aux adsorbants antérieurs.

Le procédé de l'invention est particulièrement intéressant lorsqu'il est couplé avec un procédé d'hydro-isomérisation, en ce qu'il permet un recyclage sélectif des paraffines linéaires et monobranchées.

## Description de l'invention

Le procédé de séparation de l'invention met en oeuvre au moins une unité de séparation fonctionnant par adsorption et contenant au moins un adsorbant zéolithique, lequel est mis en contact avec une charge hydrocarbonée contenant des hydrocarbures ayant de 5 à 8 atomes de carbone par molécule, notamment des paraffines linéaires, des paraffines monobranchées et des paraffines multibranchées de manière à obtenir au moins deux effluents, l'un des effluents étant riche en paraffines dibranchées, tribranchées et éventuellement en composés naphténiques et/ou aromatiques.

Dans tout ce qui suit, on entend par paraffines multibranchées des paraffines présentant au moins deux ramifications. Selon l'invention, les paraffines multibranchées incluent donc les paraffines dibranchées.

Le procédé de l'invention se caractérise en ce que ledit adsorbant présente une structure mixte avec des canaux principaux dont l'ouverture est définie par un anneau à 10 atomes d'oxygène (également appelés 10 MR) et des canaux secondaires dont l'ouverture est définie par un anneau à au moins 12 atomes d'oxygène (12 MR), les canaux à au moins 12 MR n'étant accessibles que par l'intermédiaire des canaux à 10 MR. On rappelle que les canaux à 10 MR respectivement à 12 MR, peuvent schématiquement être représentés par une succession continue d'anneaux, chaque anneau étant constitué de 10, respectivement 12, atomes d'oxygène. L'invention n'est nullement limitée à l'utilisation d'un adsorbant zéolithique présentant des canaux ayant un nombre spécifique d'anneaux. En particulier, on ne sort pas du cadre de l'invention si le procédé de séparation de paraffines multibranchées est mis en oeuvre avec un adsorbant présentant des canaux à 10 MR restreints à un seul anneau. Ces adsorbants zéolithiques peuvent présenter une structure mono-, bi- ou tridimensionnelle.

Selon l'invention, l'adsorbant zéolithique adsorbe préférentiellement les paraffines linéaires, dans une moindre mesure les paraffines monobranchées et enfin de façon minoritaire les paraffines multibranchées, les composés naphténiques et aromatiques.

La charge traitée dans le procédé selon l'invention provient de la coupe C5-C8 ou de toutes coupes intermédiaires (comme par exemple C5-C7, C6-C8, C6-C7, C7-C8, C7 ou C8) comprenant des hydrocarbures paraffiniques et éventuellement naphténiques, aromatiques et oléfiniques. Ces coupes peuvent être issues de la distillation atmosphérique du pétrole brut, d'une unité de réformage (réformat léger) ou d'une unité de conversion (naphta d'hydrocracking par exemple). Dans la suite du texte, cet ensemble de charges possibles sera désigné par les termes « coupe C5-C8 et coupes intermédiaires ».

La charge traitée dans le procédé selon l'invention est composée principalement de paraffines linéaires, monobranchées et multibranchées, de composés naphténiques, tels que les diméthylcyclopentanes, de composés aromatiques tels que le benzène ou le toluène et éventuellement de composés oléfiniques.

La charge peut notamment contenir du normal pentane, du 2-méthylbutane, du néopentane, du normal hexane, du 2-méthylpentane, du 3-méthylpentane, du 2,2-diméthylbutane, du 2,3 diméthylbutane, du normal heptane, du 2-méthylhexane, du 3-méthylhexane, du 2,2-diméthylpentane, du 3,3-diméthylpentane, du 2,3-diméthylpentane, du 2,4-diméthylpentane, du 2,2,3-triméthylbutane, du normal octane, du 2-méthylheptane, du 3-méthylheptane, du 4-méthylheptane, du 2,2-diméthylhexane, du 3,3-diméthylhexane, du 2,3-diméthylhexane, du 3,4-diméthylhexane, du 2,4-diméthylhexane, du 2,5-diméthylhexane, du 2,2,3-triméthylpentane, du 2,3,3-triméthylpentane, du 2,3,4-triméthylpentane. Dans la mesure où la charge vient des coupes C5-C8 ou de coupes intermédiaires (comme par exemple C5-C7, C6-C8, C6-C7, C7-C8, C7, C8) obtenues après distillation atmosphérique, elle peut de plus contenir des alcanes cycliques, tels que les diméthylcyclopentanes, des hydrocarbures aromatiques (tels que benzène, toluène, xylènes) ainsi que d'autres hydrocarbures C9+ (c'est à dire des hydrocarbures contenant au moins 9 atomes de carbone) en quantité moindre. Les charges C5-C8 ou celles composées de coupes intermédiaires d'origine réformat peuvent de plus contenir des hydrocarbures oléfiniques, en particulier lorsque les unités reforming sont opérées à basse pression.

La teneur en paraffines (P) dépend essentiellement de l'origine de la charge, c'est-à-dire de son caractère paraffinique ou naphténique et aromatique, parfois mesuré par le paramètre N+A (somme de la teneur en naphtènes (N) et de la teneur en aromatiques (A)), ainsi que de son point initial de distillation, c'est-à-dire de la teneur en C5 et C6 dans la charge. Dans les naphtas d'hydrocracking, riches en composés naphténiques, ou les réformats légers, riches en composés aromatiques, la teneur en paraffines dans la charge sera en général faible, de l'ordre de 30 % en masse. Dans les coupes C5-C8 ou dans les coupes intermédiaires (comme par exemple C5-C7, C6-C8, C6-C7, C7-C8, C7, C8) de distillation directe, la teneur en paraffines varie entre 30 et 80 % en masse, avec une valeur moyenne de 55-60 % en masse.

La charge traitée contenant des paraffines ayant entre 5 et 8 atomes de carbone est en général de faible indice d'octane et le procédé selon l'invention consiste à la fractionner en au moins deux effluents distincts, un de ces effluents étant riche en paraffines dibranchées, tribranchées et éventuellement en composés naphténiques et/ou aromatiques. Selon les conditions de mise en oeuvre, on peut également séparer la charge en trois effluents distincts d'indices d'octane moteur et recherche croissant, respectivement riches en paraffines linéaires, en paraffines monobranchées et en paraffines dibranchées, tribranchées et éventuellement en composés naphténiques et/ou aromatiques.

Selon l'invention, le fractionnement s'effectue dans une unité de séparation contenant un ou plusieurs adsorbants, au moins un des adsorbants étant un solide zéolihtique ayant une structure mixte dont le réseau microporeux présente à la fois des canaux principaux dont l'ouverture est définie par un anneau à 10 atomes d'oxygène (également appelés 10 MR) et des canaux secondaires dont l'ouverture est définie par un anneau à au moins 12 atomes d'oxygène (12 MR), lesdits canaux principaux et secondaires étant disposés de telle manière que l'accès aux canaux secondaires d'au moins 12 MR ne soit possible que par l'intermédiaire des canaux principaux à 10 MR.

Ces différents adsorbants ont des tailles de canaux telles que chacun des isomères des coupes C5-C8 ou des coupes intermédiaires peut être adsorbé. La cinétique de diffusion de ces isomères dans les canaux à 10 MR est cependant suffisamment différente pour être mise à profit.

Conformément à l'invention, une sélectivité diffusionnelle optimale est obtenue en freinant l'entrée des molécules multibranchées par l'intermédiaire des canaux à 10 MR et une capacité d'adsorption optimale est obtenue par la présence des canaux à au moins 12 MR.

Il va de soi que le procédé de séparation de l'invention est basé sur la différence de cinétique d'adsorption des espèces à séparer et exploite ainsi les caractéristques de la séparation dite « diffusionnelle ».

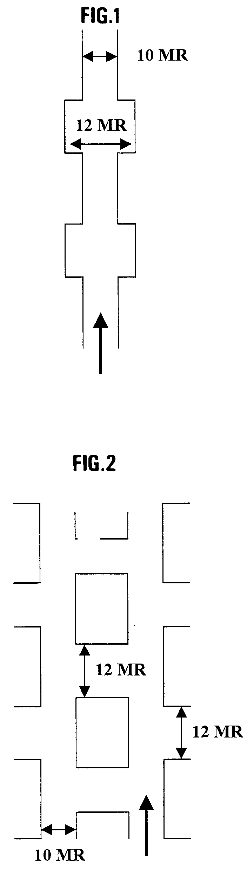

Les canaux à au moins 12 MR peuvent être soit de simples poches latérales (ou encore appelés par l'homme de l'art « side pockets») (cf. figure 1) soit former des segments poreux perpendiculaires aux canaux à 10 MR, tels que ces segments ne soient accessibles que par les canaux à 10 MR (cf. figure 2).

Les adsorbants utilisés dans la mise en oeuvre du procédé selon l'invention contiennent avantageusement du silicium et au moins un élément T choisi dans le groupe formé par l'aluminium, le fer, le gallium et le bore, de préférence l'aluminium et le bore. La teneur en silice de ces adsorbants peut être variable. Les adsorbants les plus adaptés à ce type de séparation sont ceux qui présentent des teneurs en silice élevées. Le rapport molaire Si/T est de préférence au moins égal à 10.

Lesdits adsorbants microporeux peuvent être sous forme acide, c'est-à-dire contenant des atomes d'hydrogène, ou préférentiellement échangés avec des cations alcalins ou alcalino-terreux.

Il est avantageux de mélanger aux adsorbants zéolithiques des zéolithes de type structural LTA, telles que celles décrites dans le brevet US-A-2 882 243, préférentiellement la zéolithe A. Dans la plupart de leurs formes cationiques échangées, notamment sous la forme calcium, ces zéolithes présentent un diamètre de pore de l'ordre de 5Å et possèdent de fortes capacités pour adsorber les paraffines linéaires. Mélangées avec des adsorbants zéolithiques ayant une structure telle que définie précédemment, elles peuvent permettre d'accentuer la séparation des fronts d'élution et donc permettre d'obtenir une meilleure pureté en chacun des flux enrichis obtenus.

Avantageusement, les adsorbants zéolithiques mis en oeuvre dans le procédé de l'invention sont des zéolithes de type structural EUO, NES et MWW. Des exemples de zéolithes incluses dans ces familles sont les zéolithes EU-1 (EP 42 226), ZSM-50 (US 4 640 829), TPZ-3 (US 4 695 667), NU-87 (EP 378 916), SSZ-37 (US 5 254 514), MCM-22, ERB-1(EP 293 032), ITQ-1 (US 6 004 941), PSH-3 (US 4 439 409), et SSZ-25 (EP 231 860). Les zéolithes NU-85 (US 5 385 718 et EP 462 745) et NU-86 (EP 463 768), qui ne possèdent pas de type structural déterminé, sont également avantageusement utilisées dans le procédé de l'invention.

Les zéolithes de type structural EUO (EU-1, ZSM-50, TPZ-3) ont un réseau poreux monodimensionnel. Les canaux principaux ont des ouvertures de 10 MR, et ils sont pourvus de poches latérales correspondant à une ouverture de 12 MR. La configuration de ces zéolithes de type structural EUO est celle présentée sur la figure 1.

Les zéolithes du type structural NES (NU-87 et SSZ-37) présentent un réseau bidimensionnel interconnecté. Elles possèdent dans une direction des canaux à 10 MR, reliés entre eux par des segments poreux de 12 MR, perpendiculaires aux canaux à 10 MR. Les canaux à 12 MR ne sont donc accessibles que par les canaux à 10 MR. La configuration de ces zéolithes du type structural NES est celle présentée sur la figure 2.

Il convient de préciser que la zéolithe NU-85 est une intercroissance de la zéolithe NU-87 et de la zéolithe EU-1 : chaque cristal de NU-85 comprend des bandes discrètes de NU-87 et EU-1, lesdites bandes présentant pratiquement entre elles une continuité du réseau cristallin.

La zéolithe NU-86 a un réseau poreux tridimensionnel. Dans une des dimensions se trouvent des canaux à 11 atomes d'oxygène (11 MR). Dans les deux autres dimensions se trouvent des canaux à 12 atomes d'oxygène avec des restrictions à 10. Les canaux à 12 MR ne sont accessibles que par les canaux à 10 MR. La configuration de la zéolithe NU-86 est celle présentée sur la figure 1.

Les zéolithes de type structural MWW (MCM-22, ERB-1, ITQ-1, PSH-3, SSZ-25) ont un réseau bidimensionnel non-interconnecté. Un des réseaux poreux est constitué de canaux de 10 MR, et le second de canaux de 12 MR reliés entre eux par des canaux de 10 MR, de telle manière que l'accès aux canaux de 12 MR ne puisse avoir lieu qu'à travers les canaux de 10 MR. La configuration de ces zéolithes de type structural MWW est celle présentée sur la figure 1.

Tout autre adsorbant zéolithique présentant des canaux principaux avec une ouverture de 10 atomes d'oxygène et des canaux secondaires avec une ouverture comptant plus de 12 atomes d'oxygène convient pour la mise en oeuvre du procédé de l'invention.

Le procédé de séparation de la présente invention peut utiliser les techniques de séparation par adsorption bien connues de l'homme de l'art telles que le PSA (Pressure Swing Adsorption), le TSA (Temperature Swing Adsorption) et les procédés chromatographiques (chromatographie d'élution ou contre-courant simulé par exemple) ou résulter d'une combinaison de ces techniques. Le procédé de séparation selon l'invention est aussi bien adapté à un fonctionnement en phase liquide qu'en phase gazeuse. De plus, généralement plusieurs unités de séparation (de deux à quinze) sont utilisées en parallèle et alternativement pour conduire à un procédé fonctionnant de façon continue alors que par nature le procédé de séparation suivant l'invention est discontinu.

L'unité de séparation du procédé selon l'invention utilise au moins un adsorbant ou un éluant qui peut être adsorbé ou non adsorbé. Ainsi, lorsque la charge contient la coupe C5, l'isopentane provenant de cette coupe peut soit être séparé par le procédé selon l'invention avec les paraffines monobranchées ou les paraffines multibranchées selon la mise en oeuvre choisie, soit être retiré des flux traversant le procédé à l'aide d'au moins un déisopentaniseur disposé en amont et/ou en aval de l'unité de séparation. Dans ce dernier cas, l'isopentane peut servir d'éluant permettant d'effectuer la séparation.

Plus généralement, il est avantageux de prélever une ou plusieurs fractions légères de la charge en amont de l'adsorbeur ou une ou plusieurs fractions légères des flux obtenus en sortie de l'adsorbeur enrichis soit en paraffines linéaires, soit en paraffines monobranchées, ladite ou lesdites fraction(s) légère(s) étant séparée(s) par distillation. Ces différentes fractions légères peuvent alors servir d'éluant adsorbable pour effectuer la séparation. Par exemple, un dépentaniseur peut être placé en amont et/ou en aval de l'adsorbeur dans le cas où la charge contient la coupe C5. Le mélange riche en pentane et isopentane ainsi recueilli peut alors servir d'éluant pour effectuer la séparation. La combinaison d'un déisopentaniseur et d'un dépentanieur est aussi possible. L'isopentane, le pentane ou le mélange de pentane et d'isopentane ainsi séparés peuvent servir d'éluant pour le procédé de séparation.

Le procédé de l'invention est particulièrement utile lorsqu'il est couplé avec une unité d'hydro-isomérisation. En effet, le procédé de séparation selon l'invention, produisant au moins deux effluents distincts, l'un à indice d'octane élevé et l'autre de faible indice d'octane, et intégré dans un procédé comprenant également au moins une unité d'hydroisomérisation permet d'effectuer le recyclage de l'effluent à faible indice d'octane vers l'unité d'hydroisomérisation, laquelle convertit les paraffines linéaires et monobranchées de faible indice d'octane en paraffines multibranchées à indice d'octane élevé.

Les conditions opératoires de l'unité de séparation dépendent de ou des adsorbants considérés, ainsi que du degré de pureté en chacun des flux désiré. Elles sont comprises entre 50°C et 450°C pour la température et de 0,01 à 7 MPa pour la pression. Plus précisément, si la séparation est effectuée en phase liquide, les conditions de séparation sont : 50°C à 250°C pour la température et 0,1 à 7 MPa, de préférence de 0,5 à 5 MPa, pour la pression. Si ladite séparation est effectuée en phase gazeuse, ces conditions sont : 150°C à 450°C pour la température et 0,01 à 7 MPa, de préférence de 0,1 à 5 MPa, pour la pression.

Les exemples qui suivent illustrent l'invention sans toutefois en limiter la portée.

### EXEMPLE 1 (selon l'invention)

Les adsorbants zéolithiques étudiés sont les zéolithes EU-1 (structure monodimensionnelle avec des poches latérales) et NU-87 (structure bidimensionnelle). Ces zéolithes sont sous leur forme échangées Na$^+$, c'est-à-dire que chacune des zéolithes brutes de synthèse, une fois calcinée, a subi trois échanges ioniques successifs dans une solution de NaCl 1N, à température ambiante. La zéolithe EU-1 a un r apport Si/B égal à 24 et la zéolithe NU-87 a un rapport Si/Al égal à 16.

_a) capacité d'adsorption :_

Les capacités d'adsorption de la EU-1 et la NU-87 ont été mesurées par gravimétrie à différentes températures (100 et 200°C) pour une pression partielle de 200 mbar d'isopentane (iC5) à l'aide d'une thermobalance symétrique TAG 24 de SETARAM. Avant chaque mesure d'adsorption, les solides sont régénérés pendant 4 heures à 380°C. Les résultats se trouvent dans le Tableau 1 ci-dessous

Tableau 1 :

| capacité d'adsorption des zéolithes EU-1 et NU-87 | | |
|---|---|---|
| Température (°C) | Masse d'iC5 adsorbée (mg.g$^{-1}$) avec une pression partielle d'iC5 de 200 mbar | |
| | EU-1 | NU-87 |
| 100 | 80.3 | 92.9 |
| 200 | 49.6 | 58.8 |

*b) sélectivité diffusionnelle :*

Les sélectivités diffusionnelles du normal hexane (nC6), du 2-méthylpentane (2MP) et du 2,2-diméthylbutane (2,2DMB) ont été déterminées expérimentalement par chromatographie inverse. Pour ce faire, la réponse d'un lit fixe de zéolithe à une perturbation de concentration de type « impulsionnelle » à été mesurée. Une colonne de 10 cm remplie de 1,4 g de zéolithe, maintenue à une température constante de 200°C est traversée par un débit d'azote à 1 nl/h. La pression dans la colonne est de 1 bar et on opère en phase gazeuse. Les réponses de la colonne aux injections des différents hydrocarbures ont été mesurées. Les résultats obtenus sont résumés dans le Tableau 2, sous forme du premier moment ($\mu_1$) ou temps moyen de sortie et du second moment ($\mu_2^c$) ou variance des courbes. L'analyse dite « des moments » (cf. p. 246 dans l'ouvrage de D. Ruthven «Principles of Adsorption and Adsorption Processes » , John Wiley and Sons, New York, 1984) nous enseigne que la résistance globale au transfert de matière notée R peut se calculer par l'intermédiaire de l'équation ci-dessous :

$$R = \frac{\mu_2^c}{2 \cdot \mu_1^2} \cdot \frac{L}{v}$$

où L est la longueur du lit et v la vitesse interstitielle dans le lit.
Cette résistance est également notée dans le Tableau 2.

Tableau 2

| Zéolithe | Température (°C) | hydrocarbure | $\mu_1$ (min) | $\mu_2^c$ (min$^2$) | R (min) |
|----------|-----------------|--------------|---------------|---------------------|---------|
| EU-1     | 200             | nC6          | 54.3          | 2074.1              | 5.1     |
|          |                 | 2 MP         | 20.6          | 330.1               | 5.6     |
|          |                 | 2,2 DMB      | 0             | 0                   | ∞       |
| Nu-87    | 200             | nC6          | 59.3          | 1220.5              | 2.5     |
|          |                 | 2 MP         | 40.1          | 1068.3              | 4.8     |
|          |                 | 2,2 DMB      | 13.1          | 546.1               | 22.9    |

On calcule le rapport α entre les résistances globales du 2MP et du 2,2DMB et entre les résistances globales du 2MP et du nC6 pour évaluer la sélectivité diffusionnelle des zéolithes EU-1 et NU-87 dans la séparation de ces trois hydrocarbures. Les valeurs de α ont été calculées à 200°C pour la EU-1 et la NU-87. Ces valeurs sont notées dans le Tableau 3.

Tableau 3

| Zéolithe | Température (°C) | α (2MP/22DMB) | α (2MP/nC6) |
|----------|-----------------|---------------|-------------|
| EU-1     | 200             | ∞             | 1.1         |
| NU-87    | 200             | 4.76          | 1.9         |

**Exemple 2 (comparatif) :**

On reprend les mêmes tests que ceux donnés dans l'exemple 1 et dans les mêmes conditions opératoires en utilisant comme adsorbant zéolithique la zéolithe silicalite de structure tridimensionnelle. La silicalite appartient au type structural MFI et présente uniquement des canaux de 10 MR. Elle est sous sa forme échangée Na$^+$ et présente un rapport Si/Al de 250.

*a) capacité d'adsorption :*

Tableau 4

| Température (°C) | Masse d'iC5 adsorbée (mg.g$^{-1}$) avec une pression partielle d'iC5 de 200 mbar |
|---|---|
| 100 | 47.0 |
| 200 | 24.0 |

D'après les résultats présentés dans les tableaux 1 et 4, on constate que les capacités d'adsorption des zéolithes EU-1 et NU-87 sont supérieures aux capacités d'adsorption de la silicalite aux températures étudiées. La capacité d'adsorption en iC5 est environ 1,9 fois supérieure à celle de la silicalite pour la EU-1 et de 2,2 fois pour la NU-87.

*b) sélectivité diffusionnelle :*

Tableau 5

| Zéolithe | Température (°C) | Hydrocarbure | $\mu_1$ (min) | $\mu_2^c$ (min$^2$) | R (min) |
|---|---|---|---|---|---|
| Silicalite | 200 | nC6 | 28.7 | 321.5 | 2.8 |
| | | 2 MP | 16.3 | 388.5 | 3.2 |
| | | 2,2 DMB | 7.5 | 183.0 | 13.3 |

Tableau 6

| Zéolithe | Température (°C) | α (2,2DMB/2MP) | α (2MP/nC6) |
|---|---|---|---|
| Silicalite | 200 | 4.17 | 1.2 |

D'après les résultats présentés dans les tableaux 3 et 6, on constate que les zéolithes EU-1 et NU-87 présentent des sélectivités diffusionnelles très intéressantes pour la séparation des hydrocarbures à différents degrés de branchements. Notamment, le 2,2DMB ne pénètre pas du tout dans les pores de la zéolithe EU-1 (tableau 2) dans les conditions expérimentales données ci-dessus, et la sélectivité de cette zéolithe pour la séparation du 2,2DMB et du 2MP est donc infinie, donc largement supérieure à celle de la silicalite. La zéolithe NU-87 présente à 200°C une meilleure sélectivité pour la séparation du 2,2DMB et du 2MP que la silicalite, et elle possède également une meilleure sélectivité que la silicalite pour la séparation du 2MP et du nC6.

En conclusion, les zéolithes NU-87 et EU-1 présentent une meilleure capacité d'adsorption que la silicalite et une sélectivité diffusionnelle généralement meilleure permettant de garantir un gain de productivité par rapport à un procédé de séparation des paraffines multibranchées utilisant la silicalite.

**Revendications**

1.  Procédé de séparation de paraffines multibranchées comprises dans une charge hydrocarbonée contenant des hydrocarbures ayant de 5 à 8 atomes de carbone par molécule, notamment des paraffines linéaires, monobranchées et multibranchées, comprenant la mise en contact de ladite charge avec au moins un adsorbant zéolithique, **caractérisé en ce que** ledit adsorbant présente au moins deux types de canaux, des canaux principaux dont l'ouverture est définie par un anneau à 10 atomes d'oxygène (10 MR) et des canaux secondaires dont l'ouverture est définie par un anneau à au moins 12 atomes d'oxygène (au moins 12 MR), lesdits canaux secondaires étant accessibles à la charge à séparer uniquement par lesdits canaux principaux.

2.  Procédé de séparation selon la revendication 1 **caractérisé en ce que** ledit adsorbant contient du silicium et au moins un élément T choisi dans le groupe formé par l'aluminium, le fer, le gallium et le bore, le rapport molaire Si/T étant au moins égal à 10.

**3.** Procédé de séparation selon la revendication 1 ou 2 **caractérisé en ce que** ledit adsorbant zéolithique est une zéolithe de type structural EUO.

**4.** Procédé de séparation selon la revendication 1 ou 2 **caractérisé en ce que** ledit adsorbant zéolithique est une zéolithe de type structural NES.

**5.** Procédé de séparation selon la revendication 1 ou 2 **caractérisé en ce que** ledit adsorbant zéolithique est une zéolithe de type structural MWW.

**6.** Procédé de séparation selon la revendication 1 ou 2 **caractérisé en ce que** ledit adsorbant zéolithique est la zéolithe NU-85.

**7.** Procédé de séparation selon la revendication 1 ou 2 **caractérisé en ce que** ledit adsorbant zéolithique est la zéolithe NU-86.

**8.** Procédé de séparation selon l'une des revendications 1 à 7 **caractérisé en ce que** ledit adsorbant zéolithique est mélangé avec une zéolithe de type structural LTA.

**9.** Procédé de séparation selon l'une des revendications 1 à 8 **caractérisé en ce que** ladite charge hydrocarbonée est issue de la distillation atmosphérique du pétrole brut.

**10.** Procédé de séparation selon l'une des revendications 1 à 8 **caractérisé en ce que** ladite charge hydrocarbonée est issue d'une unité de reformage.

**11.** Procédé de séparation selon l'une des revendications 1 à 8 **caractérisé en ce que** ladite charge hydrocarbonée est issue d'une unité de conversion.

**12.** Procédé de séparation selon l'une des revendications 1 à 11 **caractérisé en ce qu'**il consiste à fractionner ladite charge hydrocarbonée en au moins deux effluents distincts, l'un au moins étant riche en paraffines multibranchées et éventuellement en composés aromatiques et naphténiques.

**13.** Procédé de séparation selon l'une des revendications 1 à 11 **caractérisé en ce qu'**il consiste à fractionner ladite charge hydrocarbonée en trois effluents distincts, un effluent riche en paraffines linéaires, un effluent riche en paraffines monobranchées et un effluent riche en paraffines multibranchées et éventuellement en composés aromatiques et naphténiques.

**14.** Procédé de séparation selon l'une des revendications 1 à 13 **caractérisé en ce qu'**au moins une fraction légère est séparée par distillation en amont ou en aval de l'unité de séparation.

**15.** Procédé de séparation selon l'une des revendications 1 à 13 **caractérisé en ce que** la charge contient la coupe C5 et au moins un déisopentanisuer et/ou au moins un dépentaniseur sont disposés en amont ou en aval de l'unité de séparation.

**16.** Procédé de séparation selon l'une des revendications 14 et 15 **caractérisé en ce que** ladite fraction légère ou l'isopentane et/ou le pentane et/ou le mélange de ces deux corps servent d'éluant pour effectuer la séparation.

**17.** Procédé de séparation selon l'une des revendications 1 à 16 **caractérisé en ce que** la séparation est réalisée en phase liquide à une température comprise entre 50°C et 250°C et à une pression comprise entre 0,1 et 7 MPa.

**18.** Procédé de séparation selon l'une des revendications 1 à 16 **caractérisé en ce que** la séparation est réalisée en phase gazeuse à une température comprise entre 150°C et 450°C et à une pression comprise entre 0,01 et 7 MPa.

## FIG.1

10 MR

12 MR

## FIG.2

12 MR

12 MR

10 MR

EP 1 182 246 A1

# RAPPORT DE RECHERCHE EUROPEENNE

**Office européen des brevets**

Numéro de la demande

EP 01 40 2164

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.7) |
|---|---|---|---|
| A | EP 0 922 748 A (INST FRANCAIS DU PETROL) 16 juin 1999 (1999-06-16) * revendications 1-14 * | 1-18 | C10G25/03 C10G67/06 |
| A | FR 2 771 419 A (INST FRANCAIS DU PETROL) 28 mai 1999 (1999-05-28) * revendications 1-26 * | 1-18 | |
| A | EP 0 934 996 A (INST FRANCAIS DU PETROL) 11 août 1999 (1999-08-11) * revendications 1-17 * | 1-18 | |

DOMAINES TECHNIQUES RECHERCHES (Int.Cl.7)

C10G
C07C

Le présent rapport a été établi pour toutes les revendications

| Lieu de la recherche | Date d'achèvement de la recherche | Examinateur |
|---|---|---|
| LA HAYE | 28 novembre 2001 | Michiels, P |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

## ANNEXE AU RAPPORT DE RECHERCHE EUROPEENNE
## RELATIF A LA DEMANDE DE BREVET EUROPEEN NO.

EP 01 40 2164

La présente annexe indique les membres de la famille de brevets relatifs aux documents brevets cités dans le rapport de recherche européenne visé ci–dessus.
Lesdits members sont contenus au fichier informatique de l'Office européen des brevets à la date du
Les renseignements fournis sont donnés à titre indicatif et n'engagent pas la responsabilité de l'Office européen des brevets.

28-11-2001

| Document brevet cité au rapport de recherche | | Date de publication | Membre(s) de la famille de brevet(s) | | Date de publication |
|---|---|---|---|---|---|
| EP 0922748 | A | 16-06-1999 | FR | 2771418 A1 | 28-05-1999 |
| | | | BR | 9805035 A | 07-12-1999 |
| | | | CN | 1226547 A | 25-08-1999 |
| | | | EP | 0922748 A1 | 16-06-1999 |
| | | | JP | 11236574 A | 31-08-1999 |
| | | | US | 6156950 A | 05-12-2000 |
| FR 2771419 | A | 28-05-1999 | FR | 2771419 A1 | 28-05-1999 |
| | | | EP | 0922750 A1 | 16-06-1999 |
| | | | JP | 11228976 A | 24-08-1999 |
| EP 0934996 | A | 11-08-1999 | FR | 2774386 A1 | 06-08-1999 |
| | | | EP | 0934996 A1 | 11-08-1999 |

EPO FORM P0460

Pour tout renseignement concernant cette annexe : voir Journal Officiel de l'Office européen des brevets, No.12/82